# EUROPÄISCHE PATENTSCHRIFT

(11) **EP 0 593 990 B1**
(45) Veröffentlichungstag und Bekanntmachung des Hinweises auf die Patenterteilung: **07.07.1999**
(21) Anmeldenummer: 93116268.9
(22) Anmeldetag: 07.10.1993
(51) Int. Cl.: A61N 1/05, A61B 5/00

(54) **Implantierbarer Sauerstoffsensor für einen Herzschrittmacher**
Implantable oxygen sensor for pacemaker
Capteur d'oxygène pour implantation d'un stimulateur cardiaque

(30) Priorität: 19.10.1992 DE 4235171
(43) Veröffentlichungstag der Anmeldung: 27.04.1994
(73) Patentinhaber: Pacesetter AB, 171 95 Solna (SE)
(72) Erfinder: Holmström, Nils, Dipl.-Ing., S-175 45 Järfälla (SE); Mund, Konrad, Dr. Dipl.-Chem., D-91080 Uttenreuth (DE)
(74) Vertreter: Lettström, Richard Wilhelm

(56) Entgegenhaltungen:
- EP-A- 170 998
- EP-A- 0 117 972
- EP-A- 0 170 997
- FR-A- 2 345 170

## Beschreibung

Die Erfindung betrifft einen implantierbaren elektrochemischen Sauerstoffsensor, mit einer Meßelektrode und einer Gegenelektrode, für einen physiologisch gesteuerten Herzschrittmacher.

Für die Therapie von Herzrhythmusstörungen werden heutzutage im allgemeinen Herzschrittmacher eingesetzt. Dabei wird der Herzmuskel durch kurze Stromimpulse stimuliert, die mittels einer Reizelektrode übertragen werden. Herzschrittmacher, die mit einer konstanten Frequenz arbeiten, simulieren die Steuerung des gesunden Herzens, das seine Schlagzahl bei Bedarf ändert, nur unvollkommen. Deshalb wird eine physiologische Steuerung des Herzschrittmachers angestrebt, wozu insbesondere die Sauerstoffkonzentration im Blut oder Gewebe als Steuerparameter dient. Dabei wird dann die Frequenz der Reizimpulse der Sauerstoffkonzentration bzw. dem O₂-Partialdruck angepaßt, um das Herz bei mangelhafter Sauerstoffversorgung schneller schlagen zu lassen. Zu diesem Zweck muß die Sauerstoffkonzentration kontinuierlich gemessen werden, d.h. es wird ein implantierbarer Sauerstoffsensor benötigt.

Aus der EP-OS 0 170 998 ist ein implantierbarer Sauerstoffsensor bekannt, der zur Steuerung von Herzschrittmachern dienen kann. Dieser Sauerstoffsensor, der auf dem elektrochemischen Prinzip beruht, weist eine Meß- bzw. Arbeitselektrode, d.h. die eigentliche Sensorelektrode, sowie eine Gegenelektrode und gegebenenfalls eine Bezugselektrode auf. Beim Betrieb des Sensors werden der Meßelektrode, die insbesondere eine glatte Glaskohlenstoffelektrode ist, zyklisch zwei Potentiale aufgeprägt, und zwar ein Meßpotential und ein sogenanntes Erholungspotential. Beispielsweise wird der Sensorelektrode bei einer Zyklusdauer von 2 s jeweils für 20 ms ein Meßpotential von -1 V aufgeprägt, bezogen auf eine Ag/AgCl-Bezugselektrode. Nach der Umladung der elektrochemischen Doppelschichtkapazität fließt dabei ein kathodischer Strom, der von der Sauerstoffkonzentration im Blut abhängig ist. Während der restlichen Zyklusdauer bleibt der Sensor unbelastet (Erholungspotential: 0 V) und floatet.

Da während des Betriebs des Sensors an der Arbeitselektrode Sauerstoff verbraucht, d.h. reduziert wird und dadurch ein kathodischer Strom fließt, wird die Gegenelektrode anodisch belastet. Dies hat zur Folge, daß das Elektrodenmaterial und/oder im Elektrolyt vorhandene Substanzen, wie Wasser (unter Sauerstoffentwicklung) und Aminosäuren, oxidiert werden.

Als Gegenelektrode eines implantierten Sauerstoffsensors kann das Gehäuse der implantierten Elektronik, d.h. des Herzschrittmachers, dienen. Dieses Gehäuse besteht vorzugsweise aus Titanblech, weil dieses Material besonders körperverträglich ist. Durch den anodischen Strom wird nun das Titan oxidiert, d.h. es bildet sich eine Oxidschicht. Als Folge davon driftet das elektrochemische Potential ins Positive und damit steigt die Spannung, die für den aufgeprägten Strom erforderlich ist, laufend an und erreicht schließlich die Größe der Versorgungsspannung. Dadurch ist dann der Verstärker nicht mehr in der Lage auszusteuern, und das Sollpotential wird nicht mehr erreicht. Infolgedessen korreliert dann das Meßsignal nicht mehr zum Sauerstoffgehalt.

Es besteht auch die Möglichkeit, als Gegenelektrode einen Ring aus Platin zu verwenden, der am Sensorkabel integriert ist. Dies bedeutet aber, daß das Kabel drei Leiter enthalten muß, und zwar je einen Leiter für die Sauerstoffelektrode, die Gegenelektrode und die Bezugselektrode. Daraus resultiert dann aber eine höhere Störanfälligkeit.

Ein Herzschrittmacher mit einem Saurstoffsensor is aus EP-A-0 170 997 bekannt. Die O₂-Messelektrode besteht vorzugsweise aus glattem Glaskohlenstoff und die Reizelektrode vorzugsweise aus aktiviertem Glaskohlenstoff. Die O₂-Messelektrode und die Reizelektrode sind im Herzmuskelgewebe angeordnet. Die Reizelektrode funktioniert hier als Gegenelektrode.

Aufgabe der Erfindung ist es, bei einem implantierbaren elektrochemischen Sauerstoffsensor für einen physiologisch gesteuerten Herzschrittmacher die Gegenelektrode so zu gestalten, daß durch die im Betrieb erfolgenden Oxidationsvorgänge die Funktion nicht gestört wird.

Dies wird erfindungsgemäß dadurch erreicht, daß als Gegenelektrode eine flächige Elektrode dient, die Bestandteil des Gehäuses des Herzschrittmachers ist.

Beim implantierbaren Sauerstoffsensor nach der Erfindung dient als Material für die Gegenelektrode aktivierter Glaskohlenstoff. Dieses Material ist biokompatibel und zeichnet sich durch eine große elektrochemische Doppelschichtkapazität von etwa 0,3 F/cm² aus (siehe EP-OS 0 170 998). Aktivierter Glaskohlenstoff weist an der Oberfläche eine - durch die Aktivierung erzeugte - mikroporöse Schicht mit Poren im nm-Bereich auf (siehe DE-OS 26 13 072).

Die flächige Gegenelektrode ist vorzugsweise auf dem Gehäuse des Herzschrittmachers angeordnet. Dazu kann beispielsweise eine Scheibe aus aktiviertem Glaskohlenstoff mittels eines Leitklebstoffes auf dem Herzschrittmachergehäuse, das im allgemeinen aus Titan besteht, befestigt werden. Ein geeigneter Leitklebstoff ist beispielsweise ein Epoxidharz, das elektrisch leitendes Kohlenstoffpulver enthält.

Die Gegenelektrode weist vorzugsweise eine Fläche bis zu 10 cm² auf, vorteilhaft ist sie ca. 3 cm² groß. Bei einer Fläche von 10 cm² ist so eine Doppelschichtkapazität von ca. 3 F verfügbar. Die Dicke der Gegenelektrode beträgt vorzugsweise ca. 1 mm.

Bei einer Kapazität von 3 F und einem mittleren anodischen Strom von 1 µA würde eine Potentialänderung um 0,1 V innerhalb von ca. 80 h erreicht werden. Diese Abschätzung zeigt, daß selbst die hohe Doppelschichtkapazität von aktiviertem Glaskohlenstoff allein nicht für ein Langzeitimplantat ausreichend wäre. Beim erfindungsgemäßen Sauerstoffsensor wird die hohe Doppelschichtkapazität jedoch durch die große innere Oberfläche der porösen Schicht der Glaskohlenstoffelektrode erreicht. Dies bedeutet einerseits, daß diese Schicht durch den geringen anodischen Strom leicht oxidiert werden kann, und andererseits, daß der anodische Strom ca. 75 Jahre lang fließen muß, bis die poröse Schicht in einer Dicke von 0,1 mm oxidiert ist. Daraus ergibt sich aber, daß beim erfindungsgemäßen Sauerstoffsensor die an ein Implantat gestellten Anforderungen (geforderte Betriebsdauer bei Herzschrittmachern: 10 Jahre) erfüllt sind.

Zur experimentellen Überprüfung dieses Sachverhaltes werden Sauerstoffsensoren in In-vitro-Versuchen belastet, und dann wird das Potential der Gegenelektrode gemessen. Dabei ergibt sich folgender Potentialverlauf. Beim Sauerstoffsensor nach der Erfindung ändert sich das Potential des mit einer Scheibe aus aktiviertem Glaskohlenstoff versehenen Titangehäuses lediglich während der Einschwingphase, bleibt dann aber stabil und stellt sich auf einen Wert von ca. 0,8 V ein, gemessen gegen eine Ag/AgCl-Elektrode. Wird dagegen lediglich das Titangehäuse als Gegenelektrode verwendet, d.h. auf aktivierten Glaskohlenstoff verzichtet, so ändert sich das Potential linear mit der Zeit. Bereits nach etwa einer Woche ist das Potential so hoch, d.h. so positiv, daß die Elektronik den Sensor nicht mehr ansteuert (bei einem Herzschrittmacher beträgt die verfügbare Spannung ca. 2 V) und das Meßsignal somit nicht mehr dem Sauerstoffgehalt entspricht.

## Patentansprüche

1. Implantierbarer elektrochemischer Sauerstoffsensor, mit einer Meßelektrode und einer Gegenelektrode, für einen physiologisch gesteuerten Herzschrittmacher, wobei, als Gegenelektrode eine flächige Elektrode aus aktiviertem Glaskohlenstoff dient, die Bestandteil des Gehäuses des Herzschrittmachers ist.

2. Implantierbarer Sauerstoffsensor nach Anspruch 1, **dadurch gekennzeichnet,** daß die flächige Gegenelektrode auf dem Gehäuse des Herzschrittmachers angeordnet ist.

3. Implantierbarer Sauerstoffsensor nach Anspruch 1 oder 2 , **dadurch gekennzeichnet,** daß die Gegenelektrode bis zu 10 cm² groß ist.

4. Implantierbarer Sauerstoffsensor nach einem der Ansprüche 1 bis 3, **dadurch gekennzeichnet,** daß die Gegenelektrode eine Dicke von ca. 1 mm aufweist.

5. Implantierbarer Sauerstoffsensor nach einem oder mehreren der Ansprüche 1 bis 4, **dadurch gekennzeichnet,** daß das Gehäuse des Herzschrittmachers aus Titan besteht.

## Claims

1. Implantable electrochemical oxygen sensor, having a measuring electrode and a counter-electrode, for a physiologically controlled heart pacemaker, with a flat electrode of activated glassy carbon being used as counter-electrode, this electrode being a component part of the housing of the heart pacemaker.

2. Implantable oxygen sensor according to claim 1, characterized in that the flat counter-electrode is arranged on the housing of the heart pacemaker.

3. Implantable oxygen sensor according to claim 1 or 2, characterized in that the counter-electrode has a size of up to 10 cm².

4. Implantable oxygen sensor according to one of claims 1 to 3, characterized in that the counter-electrode has a thickness of about 1 mm.

5. Implantable oxygen sensor according to one or more of claims 1 to 4, characterized in that the housing of the heart pacemaker consists of titanium.

## Revendications

1. Capteur électrochimique d'oxygène, qui peut être implanté et qui comprend une électrode de mesure et une contre électrode, pour un stimulateur cardiaque commandé physiologiquement, qui utilise, comme contre électrode une électrode ayant de la surface, en carbone vitreux activé et qui fait partie du boîtier du stimulateur cardiaque.

2. Capteur d'oxygène pouvant être implanté suivant la revendication 1, caractérisé en ce que la contre électrode ayant de la surface est disposée sur le boîtier du stimulateur cardiaque.

3. Capteur d'oxygène pouvant être implanté suivant la revendication 1 ou 2, caractérisé en ce que la contre électrode a une surface allant jusqu'à 10 cm².

4. Capteur d'oxygène pouvant être implanté suivant une des revendications 1 à 3, caractérisé en ce que la contre électrode a une épaisseur de 1 mm environ.

5. Capteur d'oxygène pouvant être implanté suivant une ou plusieurs des revendications 1 à 4, caractérisé en ce que le boîtier du stimulateur cardiaque est en titane.
